(19) Europäisches Patentamt
European Patent Office
Office européen des brevets

(11) Veröffentlichungsnummer: **0 564 788 A2**

# (12) EUROPÄISCHE PATENTANMELDUNG

(21) Anmeldenummer: **93102325.3**

(22) Anmeldetag: **15.02.93**

(51) Int. Cl.5: **C07D 471/04**, A61K 31/435, C07D 295/22, C07D 207/48, C07D 211/96, C07D 403/04, C07F 9/6561, //(C07D471/04, 235:00,221:00)

(30) Priorität: **27.02.92 DE 4206042**

(43) Veröffentlichungstag der Anmeldung:
**13.10.93 Patentblatt 93/41**

(84) Benannte Vertragsstaaten:
**AT BE CH DE DK ES FR GB GR IE IT LI LU MC NL PT SE**

(71) Anmelder: **BAYER AG**

**D-51368 Leverkusen(DE)**

(72) Erfinder: **Hanko, Rudolf, Dr.**
**Schillerstrasse 23**
**W-4000 Düsseldorf(DE)**
Erfinder: **Dressel, Jürgen, Ph.D.**
**Claudiusweg 9**
**W-5600 Wuppertal(DE)**
Erfinder: **Fey, Peter, Dr.**
**Am Eickhof 23**
**W-5600 Wuppertal(DE)**
Erfinder: **Hübsch, Walter, Dr.**
**Am Eckbusch 43/50**
**W-5600 Wuppertal(DE)**
Erfinder: **Krämer, Thomas, Dr.**
**In den Birken 92a**
**W-5600 Wuppertal(DE)**
Erfinder: **Müller, Ulrich E., Dr.**
**Claudiusweg 9**
**W-5600 Wuppertal(DE)**
Erfinder: **Müller-Gliemann, Matthias Dr.**
**Claudiusweg 5**
**W-5600 Wuppertal(DE)**
Erfinder: **Beuck, Martin, Dr.**
**Trills 7**
**W-4006 Erkrath 2(DE)**
Erfinder: **Kazda, Stanislav, Prof.Dr.**
**Gellertweg 18**
**W-5600 Wuppertal(DE)**
Erfinder: **Hirth-Dieter, Claudia, Dr.**
**Claudiusweg 9**
**W-5600 Wuppertal(DE)**
Erfinder: **Knorr, Andreas, Dr.**
**Trillser Graben 10**
**W-4006 Erkrath 2(DE)**
Erfinder: **Stasch, Kohannes-Peter, Dr.**
**Schneewittchenweg 37**
**W-5600 Wuppertal(DE)**
Erfinder: **Wohlfeil, Stefan, Dr.**
**Tucherweg 25**
**W-4010 Hilden(DE)**
Erfinder: **Yalkinoglou, Özkan, Dr.**
**Neuer Weg 21**
**W-5600 Wuppertal(DE)**

(54) **Sulfonylbenzyl substituierte Imidazopyridine als A II-antagonisten.**

(57) Imidazopyridine der Formel (I)

EP 0 564 788 A2

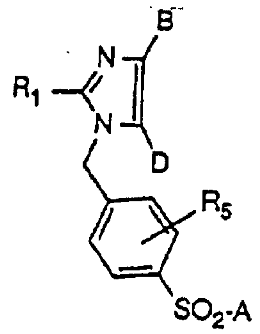

(I),

in welcher

R¹ für Alkyl oder Alkenyl steht, die gegebenenfalls durch Cycloalkyl substituiert sind, oder für Cycloalkyl steht,

B und D gemeinsam einen Rest der Formel

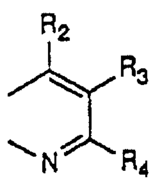

,

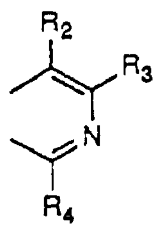

,

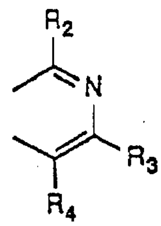

bilden,

oder

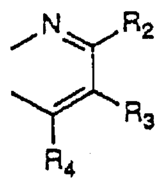

A für einen über das Stickstoffatom gebundenen 3 bis 8-gliedrigen, gesättigten Heterocyclus mit bis zu 2 weiteren Heteroatomen aus der Reihe S, N oder O steht und der gegebenenfalls bis zu 2-fach gleich oder verschieden durch Perfluoralkyl mit bis zu 5 Kohlenstoffatomen oder durch einen Rest der Formel

$-SO_3H$ ,

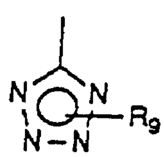

, $-CO-R^{10}$ oder $-\overset{\overset{O}{\|}}{P}(OR^{11})(OR^{12})$

substituiert ist.

Die Sulfonylbenzyl-substituierten Imidazopyridine können als Wirkstoffe in Arzneimitteln, insbesondere zur Behandlung von Hypertonie und Atherosklerose, eingesetzt werden.

Die Erfindung betrifft Sulfonylbenzyl-substituierte Imidazopyridine, ein Verfahren zu ihrer Herstellung und ihre Verwendung in Arzneimitteln, insbesondere als blutdrucksenkendes und anti-atherosklerotisches Mittel.

Es ist bekannt, daß Renin, ein proteolytisches Enzym, in vivo vom Angiotensinogen das Dekapeptid Angiotensin I abspaltet, welches wiederum in der Lunge, den Nieren oder anderen Geweben zu dem blutdrucksteigerndes Oktapeptid Angiotensin II abgebaut wird. Die verschiedenen Effekte des Angiotensin II, wie beispielsweise Vasokonstriktion, $Na^+$-Retention in der Niere, Aldosteronfreisetzung in der Nebenniere und Tonuserhöhung des sympathischen Nervensystems wirken synergistisch im Sinne einer Blutdruckerhöhung.

Darüberhinaus besitzt Angiotensin II die Eigenschaft, das Wachstum und die Vermehrung von Zellen wie beispielsweise von Herzmuskelzellen und glatten Muskelzellen zu fördern, wobei diese bei verschiedenen Krankheitszuständen (z.B. Hypertonie, Atherosklerose und Herzinsuffizienz) vermehrt wachsen und proliferieren.

Ein möglicher Ansatz zum Eingriff in das Renin-Angiotensin-System (RAS) ist neben der Inhibierung der Reninaktivität die Hemmung der Aktivität des Angiotensin-Konversionsenzyms (ACE) sowie die Blockade von Angiotensin II-Rezeptoren.

Die Erfindung betrifft Sulfonylbenzyl-substituierte Imidazopyridine der allgemeinen Formel (I)

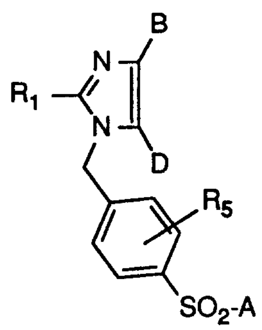

(I),

in welcher

$R^1$ für geradkettiges oder verzweigtes Alkyl oder Alkenyl mit jeweils bis zu 8 Kohlenstoffatomen steht, die gegebenenfalls durch Cycloalkyl mit 3 bis 6 Kohlenstoffatomen substituiert sind, oder
für Cycloalkyl mit 3 bis 8 Kohlenstoffatomen steht,

B und D gemeinsam einen heterocyclischen Rest der Formel

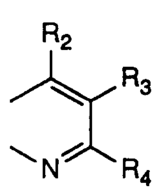 , 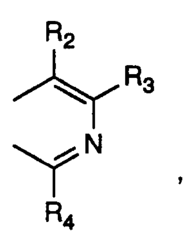 , 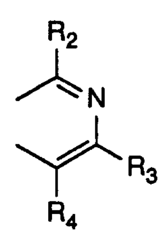

oder 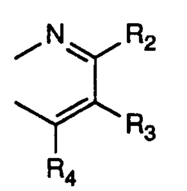

bilden,
worin

$R^2$ und $R^3$ gleich oder verschieden sind und Wasserstoff, Halogen oder geradkettiges oder verzweigtes Alkyl mit bis zu 8 Kohlenstoffatomen bedeuten,

$R^4$ die oben angegebene Bedeutung von $R^2$ und $R^3$ hat und mit diesen gleich oder verschieden ist,
oder eine Gruppe der Formel $-CO-R^6$ bedeutet,
worin

$R^6$ Hydroxy, geradkettiges oder verzweigtes Alkoxy mit bis zu 8 Kohlenstoffatomen, Phenoxy, Benzyloxy oder eine Gruppe der Formel $-NR^7R^8$ bedeutet,
worin

$R^7$ und $R^8$ gleich oder verschieden sind und Wasserstoff oder geradkettiges oder verzweigtes Alkyl mit bis zu 6 Kohlenstoffatomen oder Phenyl bedeuten,

$R^5$ für Wasserstoff, Halogen, geradkettiges oder verzweigtes Alkyl mit bis zu 8 Kohlenstoffatomen steht, oder
für geradkettiges oder verzweigtes Perfluoralkyl mit bis zu 6 Kohlenstoffatomen steht, oder
für eine Guppe der Formel -OX steht,
worin

X Wasserstoff, Benzyl, eine Hydroxyschutzgruppe oder geradkettiges oder verzweigtes Alkyl mit bis zu 8 Kohlenstoffatomen bedeutet,

A für einen über das Stickstoffatom gebundenen 3 bis 8-gliedrigen, gesättigten Heterocyclus mit bis zu 2 weiteren Heteroatomen aus der Reihe S, N oder O steht und der gegebenenfalls bis zu 2-fach gleich oder verschieden durch Perfluoralkyl mit bis zu 5 Kohlenstoffatomen oder durch einen Rest der Formel
$-SO_3H$ ,

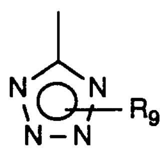 ,

$-CO-R^{10}$ oder

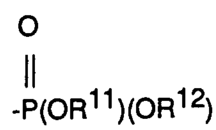

substituiert ist,
worin

$R^9$ Wasserstoff, geradkettiges oder verzweigtes Alkyl mit bis zu 6 Kohlenstoffatomen oder Triphenylmethyl bedeutet

$R^{10}$ die oben angegebene Bedeutung von $R^6$ hat und mit diesem gleich oder verschieden ist und

$R^{11}$ und $R^{12}$ gleich oder verschieden sind und Wasserstoff, geradkettiges oder verzweigtes Alkyl mit bis zu 8 Kohlenstoffatomen oder Phenyl bedeuten,

und deren Salze.

Die erfindungsgemäßen Sulfonylbenzyl-substituierten Imidazopyridine können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der Sulfonylbenzyl-substituierten Imidazopyridine können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen, sein. Besonders bevorzugt sind z.B.

Natrium-, Kalium-, Magnesium-oder Calciumsalze, sowie Ammoniumsalze, die abgeleitet sind von Ammoniak oder organischen Aminen wie beispielsweise Ethylamin, Di- bzw. Triethylamin, Di-bzw. Triethanolamin, Dicyclohexylamin, Dimethylaminoethanol, Arginin, Lysin oder Ethylendiamin.

Die erfindungsgemäßen Verbindungen können in stereoisomeren Formen, entweder als Enantiomer oder als Diastereomer, existieren. Die Erfindung betrifft sowohl die Enantiomeren oder Diastereomeren als auch deren jeweiligen Mischungen. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Ein über N gebundener, 3- bis 8-gliedriger gesättigter Heterocyclus, der außerdem als Heteroatome bis zu 2 Sauerstoff-, Schwefel- und/oder Stickstoffatome enthalten kann, steht im allgemeinen für Azetidinyl, Piperidyl, Morpholinyl, Piperazinyl oder Pyrrolidyl. Bevorzugt sind 5- und 6-gliedrige Ringe mit einem Sauerstoff-und/oder bis zu 2-Stickstoffatomen, wie beispielsweise Azetidinyl, Piperidyl, Morpholinyl oder Piperazinyl oder Pyrrolidinyl. Besonders bevorzugt sind Piperidyl und Pyrrolidinyl.

Hydroxyschutzgruppe im Rahmen der oben angegebenen Definition steht im allgemeinen für eine Schutzgruppe aus der Reihe: Trimethylsilyl, Triethylsilyl, Triisopropylsilyl, tert.Butyldimethylsilyl, tert.Butyldiphenylsilyl, Triphenylsilyl, Trimethylsilylethoxycarbonyl, Benzyl, Triphenylmethyl (Trityl), Monomethoxytrityl (MMTr), Dimethoxytrityl (DMTr), Benzyloxycarbonyl, 2-Nitrobenzyl, 4-Nitrobenzyl, 2-Nitrobenzyloxycarbonyl, 4-Nitrobenzyloxycarbonyl, tert.Butyloxycarbonyl, Allyloxycarbonyl, 4-Methoxybenzyl, 4-Methoxybenzyloxycarbonyl, Formyl, Acetyl, Trichloracetyl, 2,2,2-Trichlorethoxycarbonyl, 2,4-Dimethoxybenzyl, 2,4-Dimethoxybenzyloxycarbonyl, Methoxymethyl, Methylthiomethyl, Methoxyethoxymethyl, [2-(Trimethylsilyl)ethoxy]methyl, 2-(Methylthiomethoxy)-ethoxycarbonyl, Tetrahydropyranyl, Benzoyl, 4-Methylbenzoyl, 4-Nitrobenzoyl, 4-Fluorbenzoyl, 4-Chlorbenzoyl und 4-Methoxybenzoyl. Bevorzugt sind Acetyl, Benzyl und tert.Butyldimethylsilyl.

Bevorzugt sind Verbindungen der allgemeinen Formel (I),
in welcher

$R^1$ für geradkettiges oder verzweigtes Alkyl oder Alkenyl mit jeweils bis zu 6 Kohlenstoffatomen steht, die gegebenenfalls durch Cyclopropyl, Cyclobutyl, Cyclopentyl oder Cyclohexyl substituiert sind, oder
für Cyclopropyl, Cyclopentyl oder Cyclohexyl steht,

B und D gemeinsam einen heterocyclischen Rest der Formel

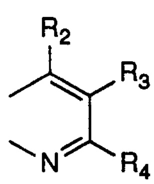

oder

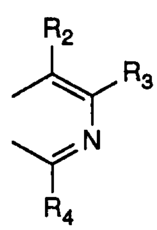

bilden,
worin

$R^2$ und $R^3$ gleich oder verschieden sind und Wasserstoff, Fluor, Chlor, Brom, geradkettiges oder verzweigtes Alkyl mit bis zu 6 Kohlenstoffatomen bedeuten,

$R^4$ die oben angegebene Bedeutung von $R^2$ und $R^3$ hat und mit diesen gleich oder verschieden ist,
oder eine Gruppe der Formel $-CO-R^6$ bedeutet,
worin

$R^6$ Hydroxy, geradkettiges oder verzweigtes Alkoxy mit bis zu 6 Kohlenstoffatomen, Phenoxy, Benzyloxy oder eine Gruppe der Formel $-NR^7R^8$ bedeutet,
worin

$R^7$ und $R^8$ gleich oder verschieden sind und Wasserstoff oder geradkettiges oder verzweigtes Alkyl mit bis zu 4 Kohlenstoffatomen bedeuten,

$R^5$ für Wasserstoff, Fluor, Chlor, Brom, geradkettiges oder verzweigtes Alkyl mit bis zu 6 Kohlenstoffatomen, oder
für geradkettiges oder verzweigtes Perfluoralkyl mit bis zu 4 Kohlenstoffatomen steht,
oder
für eine Guppe der Formel -OX steht,

worin

X Wasserstoff, Benzyl, Acetyl oder geradkettiges oder verzweigtes Alkyl mit bis zu 6 Kohlenstoffatomen bedeutet,

A für über das Stickstoffatom gebundenes Azetidinyl, Piperidyl, Pyrrolidinyl oder Morpholinyl steht, die gegebenenfalls durch Trifluormethyl oder durch einen Rest der Formel $-SO_3H$ ,

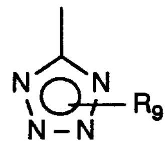
,

$-CO-R^{10}$ oder

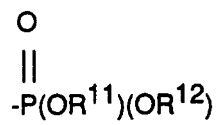

substituiert sind,
worin

$R^9$ Wasserstoff, geradkettiges oder verzweigtes Alkyl mit bis zu 4 Kohlenstoffatomen oder Triphenylmethyl bedeutet

$R^{10}$ die oben angegebene Bedeutung von $R^6$ hat und mit diesem gleich oder verschieden ist und

$R^{11}$ und $R^{12}$ gleich oder verschieden sind und Wasserstoff, geradkettiges oder verzweigtes Alkyl mit zu 6 Kohlenstoffatomen oder Phenyl bedeuten,

und deren Salze.

Besonders bevorzugt sind Verbindungen der allgemeinen Formel (I),
in welcher

$R^1$ für geradkettiges oder verzweigtes Alkyl oder Alkenyl mit jeweils bis zu 4 Kohlenstoffatomen oder Cyclopropyl steht,

B und D gemeinsam einen heterocyclischen Rest der Formel

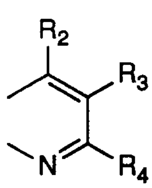
oder
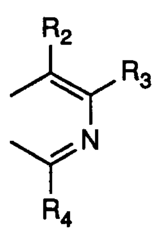

bilden,
worin

$R^2$ und $R^3$ gleich oder verschieden sind und Wasserstoff, Fluor, Chlor, Brom oder geradkettiges oder verzweigtes Alkyl mit bis zu 4 Kohlenstoffatomen bedeuten,

$R^4$ die oben angegebene Bedeutung von $R^2$ und $R^3$ hat und mit diesen gleich oder verschieden ist,
oder eine Gruppe der Formel $-CO-R^6$ bedeutet,
worin

$R^6$ Hydroxy, geradkettiges oder verzweigtes Alkoxy mit bis zu 4 Kohlenstoffatomen, Phenoxy, Benzyloxy oder eine Gruppe der Formel $-NR^7R^8$ bedeutet,
worin

$R^7$ und $R^8$ gleich oder verschieden sind und Wasserstoff oder geradkettiges oder verzweigtes Alkyl mit bis zu 3 Kohlenstoffatomen bedeuten,

$R^5$ für Wasserstoff, Fluor, Chlor, geradkettiges oder verzweigtes Alkyl mit bis zu 4 Kohlenstoffatomen, oder
für geradkettiges oder verzweigtes Perfluoralkyl mit bis zu 3 Kohlenstoffatomen steht, oder
für eine Guppe der Formel -OX steht,
worin

X Wasserstoff, Benzyl, Acetyl oder geradkettiges oder verzweigtes Alkyl mit bis zu 6 Kohlenstoffatomen bedeutet,

A für über das Stickstoffatom gebundenes Azetidinyl, Piperidyl oder Pyrrolidinyl steht, die gegebenenfalls durch Trifluormethyl oder durch einen Rest der Formel
$-SO_3H$ ,

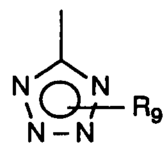 ,

$-CO-R^{10}$ oder

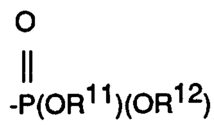

substituiert sind,
worin

$R^9$ Wasserstoff, Methyl, Ethyl oder Triphenylmethyl bedeutet

$R^{10}$ die oben angegebene Bedeutung von $R^6$ hat und mit diesem gleich oder verschieden ist und

$R^{11}$ und $R^{12}$ gleich oder verschieden sind und Wasserstoff, geradkettiges oder verzweigtes Alkyl mit bis zu 4 Kohlenstoffatomen oder Phenyl bedeuten,

und deren Salze.

Außerdem wurde ein Verfahren zur Herstellung der erfindungsgemäßen Verbindungen der allgemeinen Formel (I) gefunden, dadurch gekennzeichnet,
daß man Sulfonylbenzylverbindungen der allgemeinen Formel (II)

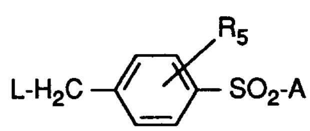 (II),

in welcher

A und $R^5$ die oben angegebene Bedeutung haben

und

L für Halogen, vorzugsweise für Brom steht,

mit Imidazopyridinen der allgemeinen Formel (III)

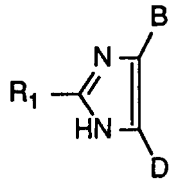

(III),

in welcher

R[1], B und D die oben angegebene Bedeutung haben,

in inerten Lösemitteln, in Anwesenheit einer Base umsetzt,

und

sowohl die Substituenten R[1] und R[5], als auch die der heterocyclischen Ringe (A, B und D), gegebenenfalls nach üblichen Methoden, beispielsweise durch Alkylierung oder Verseifung, variiert.

Das erfindungsgemäße Verfahren kann durch folgendes Formelschema beispielhaft erläutert werden:

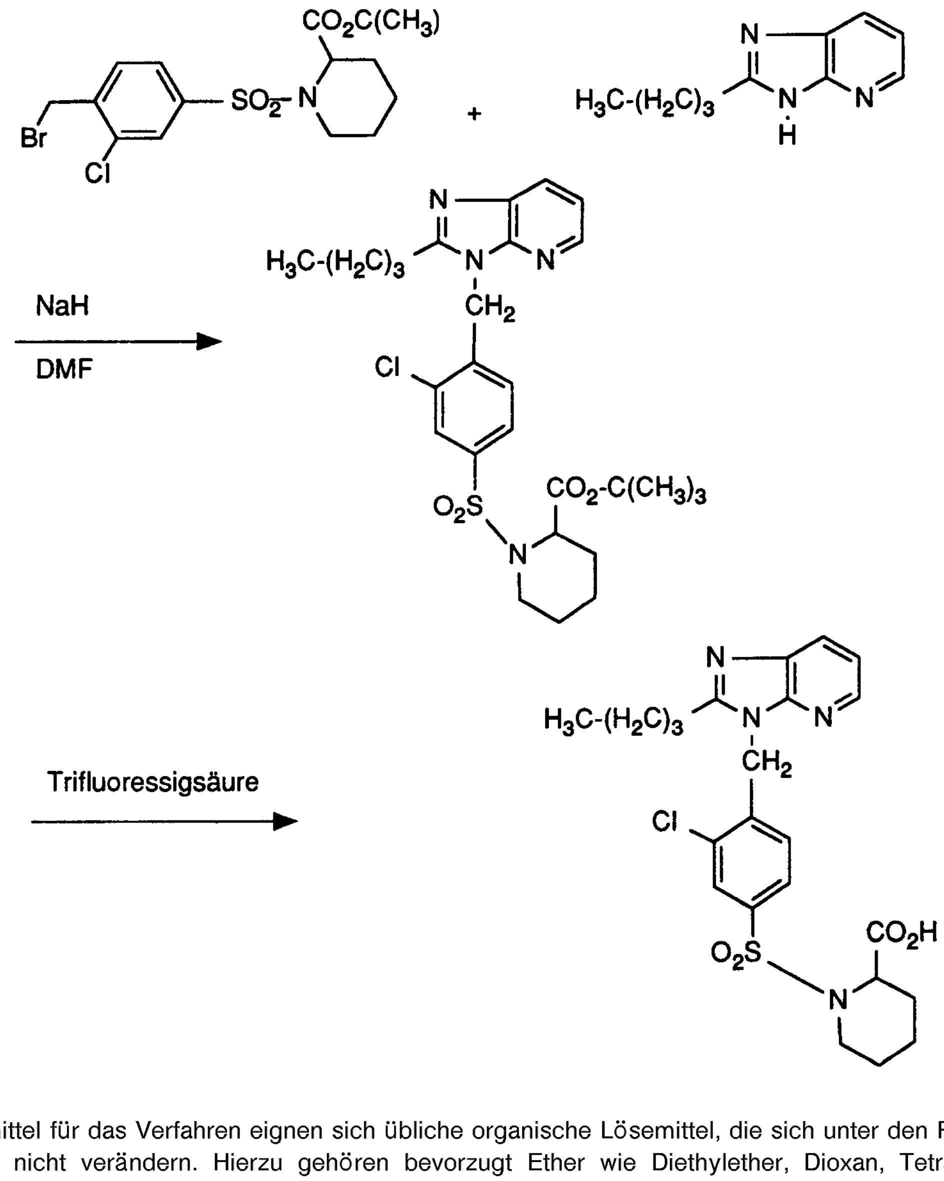


Als Lösemittel für das Verfahren eignen sich übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind für die verschiedenen Schritte Tetrahydrofuran, Methylenchlorid, Toluol oder Dimethylformamid.

Als Basen für das erfindungsgemäße Verfahren können im allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkali- oder Erdalkalialkoholate oder -amide wie Natrium-oder Kaliummethanolat, Natrium- oder Kaliumethanolat oder Kalium-tert.butylat oder Lithiumdiisopropylalmid (LDA), oder organische Amine (Trialkyl($C_1$-$C_6$)amine) wie Triethylamin, oder Heterocyclen wie 1 ,4-Diazabicyclo-[2.2.2]octan (DABCO), 1,8-Diazabicyclo[5.4.0]undec-7-en (DBU), Pyridin, Diaminopyridin, Methylpiperidin oder Morpholin. Es ist auch möglich, als Basen Alkalimetalle, wie Natrium oder deren Hydride wie Natriumhydrid, einzusetzen. Bevorzugt sind Natriumhydrid, Lithiumdiisopropylamid (LDA) und DBU.

Im allgemeinen setzt man die Base in einer Menge von 0,05 mol bis 10 mol, bevorzugt von 1 mol bis 2 mol bezogen auf 1 mol der Verbindung der Formel (III) ein.

Das erfindungsgemäße Verfahren wird im allgemeinen in einem Temperaturbereich von -100 °C bis +100 °C, bevorzugt von 0 °C bis +30 °C durchgeführt.

Das erfindungsgemäße Verfahren wird im allgemeinen bei Normaldruck durchgeführt. Es ist aber auch möglich, das Verfahren bei Überdruck oder bei Unterdruck durchzuführen (z.B. in einem Bereich von 0,5 bis 5 bar).

Als Basen eignen sich für die Verseifung die üblichen anorganischen Basen. Hierzu gehören bevorzugt Alkalihydroxide oder Erdalkalihydroxide wie beispielsweise Natriumhydroxid, Katiumhydroxid oder Bariumhydroxid, oder Alkalicarbonate wie Natrium- oder Kaliumcarbonat oder Natriumhydrogencarbonat, oder Alkalialkoholate wie Natriummethanolat, Natriumethanolat, Kaliummethanolat, Kaliumethanolat oder Kalium-tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid, oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung kann auch mit Säuren wie beispielsweise Trifluoressigsäure, Essigsäure, Chlorwasserstoffsäure, Bromwasserstoffsäure, Methansulfonsäure, Schwefelsäure oder Perchlorsäure, bevorzugt mit Trifluoressigsäure erfolgen.

Die Verseifung wird im allgemeinen in einem Temperaturbereich von 0 °C bis +100 °C, bevorzugt von +20 °C bis +80 °C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base im allgemeinen in einer Menge von 1 bis 3 mol, bevorzugt von 1 bis 1,5 mol bezogen auf 1 mol des Esters, eingesetzt. Besonders bevorzugt verwendet man molare Mengen der Reaktanden.

Bei der Durchführung der Reaktion entstehen im ersten Schritt die Carboxylate der erfindungsgemäßen Verbindungen als Zwischenprodukte, die isoliert werden können. Die erfindungsgemäßen Säuren erhält man durch Behandeln der Carboxylate mit üblichen anorganischen Säuren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren hierbei als vorteilhaft erwiesen, die basische Reaktionsmischung der Verseifung in einem zweiten Schritt ohne Isolierung der Carboxylate anzusäuern. Die Säuren können dann in üblicher Weise isoliert werden. Im Fall der basischen Heterocyclen können durch das Behandeln der Losungen der Carboxylate mit den oben aufgeführten Säuren auch die Salze der Heterocyclen mit den anorganischen Säuren gewonnen werden.

Die Alkylierung erfolgt im allgemeinen mit Alkylierungsmitteln wie beispielsweise ($C_1$-$C_6$)-Alkylhalogeniden, Sulfonsäureestern oder substituierten oder unsubstituierten ($C_1$-$C_6$)-Dialkyl- oder ($C_1$-$C_6$)-Diarylsulfonaten, vorzugsweise Methyljodid oder Dimethylsulfat.

Die Alkylierung erfolgt im allgemeinen in einem der oben aufgeführten Lösemitteln, vorzugsweise in Dimethylformamid in einem Temperaturbereich von 0 °C bis +70 °C, vorzugsweise von 0 °C bis +30 °C und Normaldruck.

Die Verbindungen der allgemeinen Formel (II) sind neu und können beispielsweise hergestellt werden, indem man, im Fall von $R^5 \neq$ -OX

Verbindungen der allgemeinen Formel (IV)

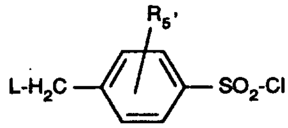

(IV),

in welcher

L die oben angegebene Bedeutung hat,

und

$R^{5'}$ die oben angegebene Bedeutung hat,

mit Verbindungen der allgemeinen Formel (V)

A-H (V),

in welcher

A die oben angegebene Bedeutung hat,

in einem der oben aufgeführten Lösemitteln in Basen, vorzugsweise in Dichlormethan mit Triethylamin bei Normaldurck und in einem Temperaturbereich von 40 ° C bis +120 ° C, vorzugsweise bei 0 ° C umsetzt,

und im Fall, daß $R^5$ für die Gruppe der Formel -OX steht,

ausgehend von Carboxy, Hydroxy-disubstituierten Benzolsulfonsäurechloriden, beispielsweise 4-Carboxy-3-hydroxybenzolsulfochlorid, zunächst durch Umsetzung mit Verbindungen der allgemeinen Formel (V), Verbindungen der allgemeinen Formel (VI) (x = H),

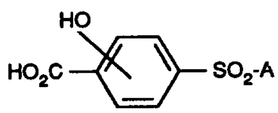

(VI)

in welcher

A die oben angegebene Bedeutung hat,

herstellt,

anschließend durch Überführung der Carboxyfunktion in den entsprechendn Benzylester und der Blockierung der Hydroxyfunktion nach üblichen Methoden in die Verbindungen der allgemeinen Formel (VII)

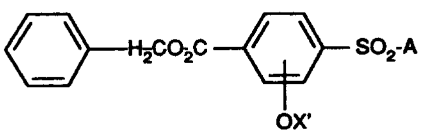

(VII)

in welcher

A die oben angegebene Bedeutung hat

und

X' die oben angegebene Bedeutung von X hat, aber nicht für Wasserstoff steht,

überführt,

in einem weiteren Schritt, ebenfalls nach bekannten Methoden, vorzugsweise mit Natriumborhydrid/LiCl in Diglyme den Benzylester zur Hydroxymethylfunktion reduziert,

und abschließend mit Triphenylphosphindibromid, in einem der oben aufgeführten Lösemittel, vorzugsweise Dimethylformamid, unter Schutzgasatmosphähre, in einem Temperaturbereich von 0 ° C bis Raumtemperatur, bromiert.

Die Verbindungen der allgemeinen Formeln (VI) und (VII) sind an sich neu und können wie oben beschrieben hergestellt werden.

Die Verbindungen der allgemeinen Formeln (IV) und (V) sind bekannt oder können nach üblicher Methode hergestellt werden.

Die Verbindungen der allgemeinen Formel (III) sind ebenfalls an sich bekannt.

Die vorstehenden Herstellungsverfahren sind lediglich zur Verdeutlichung angegeben. Die Herstellung der erfindungsgemäßen Verbindungen der allgemeinen Formel (I) ist nicht auf diese Verfahren beschränkt

jede Modifikation dieser Verfahren ist in gleicher Weise für die Herstellung anwendbar.

Die erfindungsgemäßen Verbindungen der allgemeinen Formel (I) zeigen ein nicht vorhersehbares, wertvolles pharmakologisches Wirkspektrum.

Die erfindungsgemäßen Verbindungen besitzen eine spezifische A II-antagonistische Wirkung, da sie die Bindung von Angiotensin II an A II-Rezeptoren hemmen. Sie unterdrücken die vasokonstriktorischen und Aldosteronsekretionsstimulierenden Effekte des Angiotensin II. Darüberhinaus inhibieren sie die Proliferation von glatten Muskelzellen.

Sie können deshalb in Arzneimitteln zur Behandlung der arteriellen Hypertonie und Atherosklerose eingesetzt werden. Darüber hinaus können sie zur Behandlung von coronaren Herzerkrankungen, Herzinsuffizienz, Störungen der Hirnleistung, ischämischen Gehirnerkrankungen, peripherer Durchblutungsstörungen, Funktionsstörungen der Niere und Nebenniere, bronchospastischen und vaskulär bedingten Erkrankungen der Atemwege, Natriumretention und Ödemen eingesetzt werden.

Darüber hinaus besitzen die Substanzen natriuretische und diuretische Wirkung. Diese Wirkung äußert sich in einer Ödemausschwemmung bei pathologischer Flüssigkeitsvermehrung kardialen und nichtkardialen Ursprungs.

Untersuchung auf die Hemmung der mit Agonisten induzierten Kontraktion

Kaninchen beiderlei Geschlechts werden durch Nackenschlag betäubt und entblutet, oder fallweise mit Nembutal (ca. 60 - 80 mg/kg i.v.) narkotisiert und durch Öffnung des Thorax getötet. Die Thoraxaorta wird entnommen, von anhaftendem Bindegewebe befreit, in 1,5 mm breite Ringsegmente geteilt und einzeln unter einer Anfangsbelastung von ca. 3,5 g in 10 ml Organbäder mit auf 37°C temperierter, Carbogenbegaster Krebs-Henseleit-Nährlösung folgender Zusammensetzung: 119 mmol/l NaCl; 2,5 mmol/l $CaCl_2$ x 2 $H_2O$; 1,2 mmol/l $KH_2PO_4$; 10 mmol/l Glucose; 4,8 mmol/l KCl; 1,4 mmol/l $MgSO_4$ x 7 $H_2O$ und 25 mmol/l $NaHCO_3$ verbracht.

Die Kontraktionen werden isometrisch durch Statham UC2-Zellen über Brückenverstärker (ifd Mülheim bzw. DSM Aalen) erfaßt und mittels A/D-Wandler (System 570, Keithley München) digitalisiert sowie ausgewertet. Die Durchführung von Agonistdosiswirkungskurven (DWK) erfolgt stündlich. Mit jeder DWK werden 3 bzw. 4 Einzelkonzentrationen im 4 min-Abstand in die Bäder appliziert. Nach Ende der DWK und darauffolgender Auswaschzyklen (16 mal jeweils ca. 5 sec/min mit der o.a. Nährlösung) schließt sich eine 28-minütige Ruhe- bzw. Inkubationsphase an, innerhalb derer die Kontraktionen in der Regel wieder den Ausgangswert erreichen.

Die Höhe der im Normalfall 3. DWK wird als Bezugsgröße für die Bewertung der in weiteren Durchgängen zu untersuchenden Testsubstanz verwendet, die bei den nachfolgenden DWK's in jeweils steigender Dosierung mit Beginn der Inkubationszeit in die Bäder appliziert wird. Jeder Aortenring wird dabei ganztägig mit immer dem gleichen Agonisten stimuliert.

| Agonisten und ihre Standardkonzentrationen (Applikationsvolumen pro Einzelgabe = 100 µl): | | |
|---|---|---|
| KCl | 22,7;32,7;42,7;52,7 | mmol/l |
| 1Noradrenalin | $3x10^{-9}$;$3x10^{-8}$;$3x10^{-7}$;$3x10^{-6}$ | g/ml |
| Serotonin | $10^{-8}$;$10^{-7}$;$10^{-6}$;$10^{-5}$ | g/ml |
| B-HT 920 | $10^{-7}$;$10^{-6}$;$10^{-5}$ | g/ml |
| Methoxamin | $10^{-7}$;$10^{-6}$;$10^{-5}$ | g/ml |
| Angiotensin II | $3x10^{-9}$;$10^{-8}$;$3x10^{-8}$;$10^{-7}$ | g/ml |

Für die Berechnung der $IC_{50}$ (Konzentration bei der die zu untersuchende Substanz eine 50%ige Hemmung verursacht) wird der Effekt jeweils an der 3. = submaximalen Agonistkonzentration zugrundegelegt.

Die erfindungsgemäßen Verbindungen hemmen die durch Angiotensin II induzierte Kontraktion der isolierten Kaninchenaorta dosenabhängig. Die durch Kalium-Depolarisation oder andere Agonisten induzierte Kontraktion wurde nicht oder in hohen Konzentrationen nur schwach gehemmt.

Tabelle A:

Hemmung der Gefäßkontraktion an isolierten Aortenringen von Kaninchen in vitro

$IC_{50}$ [nM] gegen Kontraktionen, induziert durch:

| Bsp.Nr.: | AII |
|---|---|
| 5 | 40 |
| 2 | 326 |
| 19 | 6 |
| 20 | 6 |
| 31 | 4 |
| 39 | 3 |
| 41 | 3 |

Blutdruckmessungen an der Angiotensin II-infundierten Ratte

Männliche Wistar Ratten (Moellegaard, Kopenhagen, Dänemark) mit einem Körpergewicht von 300 - 350 g, werden mit Thiopental (100 mg/kg i.p.) anästhesiert. Nach Tracheotomie wird in die Femoralarterie ein Katheter zur Blutdruckmessung und in die Femoralvenen ein Katheter für die Angiotensin II-Infusion und ein Katheter für die Substanzverabreichung eingeführt. Nach Gabe des Ganglienblockers Pentolinium (5 mg/kg i.v.) wird die Angiotensin II-Infusion (0,3 µg/kg/min) gestartet. Sobald die Blutdruckwerte ein stabiles Plateau erreicht haben, werden die Testsubstanzen entweder intravenös oder als Suspension bzw. Lösung in 0,5% Tylose oral verabreicht. Die Blutdruckveränderungen unter Substanzeinfluß sind als Mittelwerte ± SEM in der Tabelle angegeben.

| Beispiel Nr. | Dosis [mg/kg]p.o. | ΔP [mm/Hg] |
|---|---|---|
| 20 | 0,1 | -48 |
| 31 | 0,1 | -62 |
| 39 | 0,1 | -32 |

Bestimmung der antihypertensiven Wirksamkeit bei wachen hypertensiven Ratten

Die orale antihypertensive Wirksamkeit der erfindungsgemäßen Verbindungen wurde an wachen Ratten mit chirurgisch induzierter unilateraler Nierenarterienstenose geprüft. Dazu wurde die rechte Nierenarterie mit einem Silberclip von 0,18 mm lichter Weite eingeengt. Bei dieser Hypertonieform ist die Plasmareninaktivität in den ersten sechs Wochen nach dem Eingriff erhöht. Der arterielle Blutdruck diese Tiere wurde in definierten Zeitabständen nach Substanzgabe mit der "Schwanzmanschette" unblutig gemessen. Die zu prüfenden Substanzen wurden aufgeschwemmt in einer Tylosesuspension intragastral ("oral") per Schlundsonde in verschiedenen Dosen appliziert Die erfindungsgemäßen Verbindungen senken den arteriellen Blutdruck der Hochdruckratten in klinisch relevanter Dosierung.

Außerdem hemmen die erfindungsgemäßen Verbindungen die spezifische Bindung von radioaktivem Angiotensin II konzentrationsabhängig.

Interaktion der erfindungsgemäßen Verbindungen mit dem Angiotensin II-Rezeptor an Membranfraktionen der Nebennierenrinde (Rind)

Nebennierenrinden vom Rind (NNR), die frisch entnommen und sorgfältig von Mark von Kapsel befreit sind, werden in Sucrose-Lösung (0,32 M) mit Hilfe eines Ultra-Turrax (Janke & Kunkel, Staufen i.B.) zu grobem Membran-Homogenat zerkleinert und in zwei Zentrifugationsschritten zu Membranfraktionen partiell

aufgereinigt.
Die Untersuchungen zur Rezeptor-Bindung werden an partiell gereinigten Membranfraktionen boviner NNR mit radioaktivem Angiotensin II in einem Assay-Volumen von 0,25 ml durchgeführt, das im einzelnen die partiell gereinigten Membranen (50 - 80 µg), $^3$H-Angiotensin II (3-5 nM), Test-Pufferlösung (50 mM Tris, pH 7,2), 5 mM $MgCl_2$ sowie die zu untersuchenden Substanzen enthält. Nach einer Inkubationszeit von 60 min bei Raumtemperatur wird die nicht gebundene Radioaktivität der Proben mittels angefeuchteter Glasfaserfilter (Whatman GF/C) separiert und die gebundene Radioaktivität nach Waschung des Proteins mit eiskalter Pufferlösung (50 mM Tris/HCl, pH 7,4, 5% PEG 6000) in einem Szintillationscocktail spektrophotometrisch gemessen. Die Analyse der Rohdaten erfolgte mit Computer-Programmen zu $K_i$- bzw. $IC_{50}$-Werten ($K_i$: für die verwendete Radioaktivität korrigierte $IC_{50}$-Werte; $IC_{50}$-Werte: Konzentration bei der die zu untersuchende Substanz eine 50%ige Hemmung der spezifischen Bindung des Radioliganden bewirkt).

| | |
|---|---|
| Bsp. 5 Ki = | 550 nM |
| Bsp. 2 Ki = | 1000 nM |

<u>Untersuchung zur Inhibition der Proliferation glatter Muskelzellen durch die erfindungsgemäßen Verbindungen</u>

Zur Feststellung der antiproliferativen Wirkung der Verbindungen werden glatte Muskelzellen verwendet, die aus den Aorten von Ratten oder Schweinen durch die Media-Explantat-Technik gewonnen werden [R. Ross, J. Cell. Biol. 50, 172, 1971].

Die Zellen werden in geeigneten Kulturschalen, in der Regel 24-Loch-Platten, ausgesät und für 2 - 3 Tage in Medium 199 mit 7,5% FCS und 7,5% NCS, 2 mM L-Glutamin und 15 mM HEPES, pH 7,4 in 5% $CO_2$ bei 37°C kultiviert. Danach werden die Zellen durch Serumentzug für 2 - 3 Tage synchronisiert und sodann mit AII, Serum oder anderen Faktoren zum Wachstum angeregt. Gleichzeitig werden Testverbindungen zugesetzt. Nach 16 - 20 Stunden wird 1 µCi $^3$H-Thymidin zugefügt und nach weiteren 4 Stunden der Einbau dieser Substanz in die TCA-präzipitierbare DNA der Zellen bestimmt.

| Bsp. Nr. | %Hemmung bei $10^{-6}$ M |
|---|---|
| 5 | 70 |
| 2 | 40 |

<u>Prüfung auf natriuretische Wirkung</u>

Nüchterne Wistar-Ratten werden mit Prüfsubstanz (suspendiert in Tylose-Lösung) oral behandelt. Anschließend wird in Diurese-Käfigen die Harnausscheidung über 6 Stunden gesammelt. Die Konzentration von Natrium und Kalium im Harn wird flammenphotometrisch bestimmt.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nichttoxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu

verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

Laufmittelgemische:

| | |
|---|---|
| a = Methylenchlorid/Methanol | 10:1 |
| b = Petrolether/Essigester | 1:1 |
| c = Essigester/Petrolether | 2:1 |
| d = Methylenchlorid/Methanol | 4:1 |
| e = Toluol/Methanol | 10:1 |

Ausgangsverbindungen

Beispiel I

4-(Brommethyl)benzol-sulfochlorid

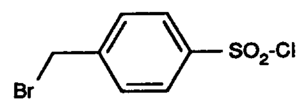

38,1 g (0,2 mol) 4-Methylbenzolsulfonylchlorid werden in 300 mi Tetrachlorkohlenstoff gelöst und mit 35,6 g (0,2 mol) N-Bromsuccinimid versetzt und nach Zugabe von 0,2 g (1,2 mmol) Azobisisobutyronitril (ABU) für 4 h unter Rückfluß erhitzt. Nach dem Abkühlen werden die Feststoffe abfiltriert und das Filtrat vom Lösemittel befreit. Flash-Chromatographie (Petrolether / Toluol 4:1, 50 $\mu$m Korngröße) und anschließende Umkristallisation aus 100 ml Cyclohexan ergibt 24,0 g (45% der Theorie) der Titelverbindung.
$R_f$ = 0,75 (Toluol)

Beispiel II

4-(Brommethyl)-3-chlorbenzolsulfochlorid

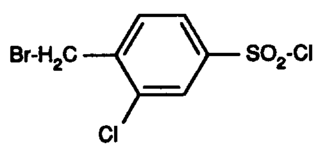

45,9 g (0,2 mol) 3-Chlor-4-methylbenzolsulfonsäure Natriumsalz werden mit 83,3 g (0,4 mol) Phosphorpentachlorid gemischt und 30 min bei 140 °C Ölbadtemperatur erhitzt. In der Hitze wird mit 500 ml Toluol versetzt, die entstandene Lösung bis zum Sieden erhitzt und nach dem Abkühlen auf Eis gegeben. Die organische Phase wird abgetrennt und mit Wasser gewaschen (2 x 200 ml). Nach dem Trocknen über $MgSO_4$ wird filtriert und alles Flüchtige im Vakuum abgezogen. Der erhaltene Rückstand wird durch

Flashchromatographie (Petrolether / Toluol 4:1, 50 µ Korngröße) gereinigt. Man erhält 24,9 g eines Produkts das sofort weiter umgesetzt wird:
Man nimmt in 200 ml Tetrachlorkohlenstoff auf und erhitzt nach Zugabe von 19,6 g (0,11 mol) N-Bromsuccinimid und 0,1 g (0,6 mmol) ABN für 6 h unter Rückfluß. Nach dem Abkühlen werden die Feststoffe abfiltriert und das Filtrat vom Lösemittel befreit. Flashchromatographie (Petrolether / Toluol 4:1, 50 µ Korngröße) ergibt 21,2 g (35%) der Titelverbindung.
$R_f$ = 0,32 (Petrolether / Dichlormethan 4:1)

Beispiel III

4-(Brommethyl)-benzolsulfonyl-N-pyrrolidinid

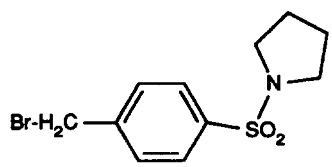

5,3 g (0,02 mol) der Verbindung aus Beispiel I werden in 200 ml Dichlormethan und 4,0 g (0,04 mol) Triethylamin gelöst und nach Zugabe von 1,4 g (0,02 mol) Pyrrolidin in 50 ml Dichlormethan bei 0°C für 1 h bei 0°C nachgerührt. Man extrahiert mit 2 N HCl (2 x 100 ml), $H_2O$ (2 x 100 ml), trocknet über $MgSO_4$, filtriert und verdampft alle flüchtigen Anteile im Vakuum.
Ausbeute: 5,4 g (89% der Theorie)
$R_f$ = 0,09 (Toluol)

Beispiel IV

4-(Brommethyl)benzolsulfonyl-N-piperidinid

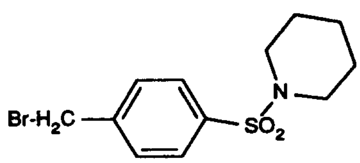

In Analogie zur Vorschrift des Beispiels III erhält man aus 1,1 g (4 mmol) der Verbindung aus Beispiel I und 0,34 g (4 mmol) Piperidin 1,0 g (81% der Theorie) der Titelverbindung.
$R_f$ = 0,14 (Toluol)

Beispiel V

(S)-4-(Brommethyl)-benzolsulfonyl-N-2-(tert.butoxycarbonyl)pyrrolidinid

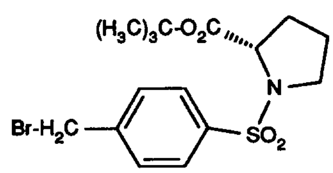

In Analogie zur Vorschrift des Beispiels III erhält man aus 7,25 g (27 mmol) der Verbindung aus Beispiel I und 4,6 g (27 mmol) S-Prolin-tert.butylester 9,1 g (84% der Theorie) der Titelverbindung.
$R_f$ = 0,66 (Petrolether / Essigester 7:3)

Beispiel VI

rac-4-(Brommethyl)-benzolsulfonyl-N-2-(tert.butoxycarbonyl)piperidinid

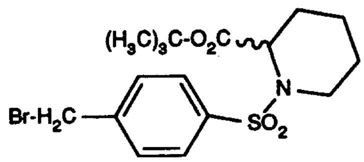

In Analogie zur Vorschrift des Beispiels III erhält man aus 8,0 g (30 mmol) der Verbindung aus Beispiel I und 5,5 g (30 mmol) rac-Pipecolinsäure-tert.butylester7,4 g (59% der Theorie) der Titelverbindung.
$R_f$ = 0,53 (Petrolether / Essigester 5:1)

Beispiel VII

(S)-4-(Brommethyl)-3-chlorbenzolsulfonyl-N-2-(tert.butoxycarbonyl)pyrrolidinid

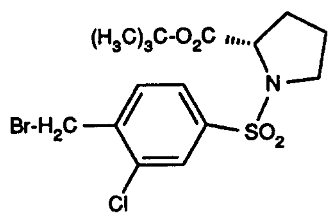

In Analogie zur Vorschrift des Beispiels III erhält man aus 10,0 g (33 mmol) der Verbindung aus Beispiel II und 5,7 g (33 mmol) S-Prolin-tert.butylester 13,9 g (96% der Theorie) der Titelverbindung.
$R_f$ = 0,55 (Petrolether / Essigester 7:3)

Beispiel VIII

rac-4-(Brommethyl)-3-chlorbenzolsulfonyl-N-2-(tert.butoxycarbonyl)piperidinid

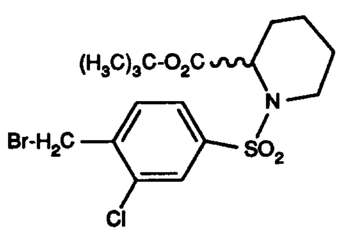

In Analogie zur Vorschrift des Beispiels III erhält man aus 10,0 g (33 mmol) der Verbindung aus Beispiel II und 6,1 g (33 mmol) rac-Pipecolinsäure-tert.butylester 14,6 g (98% der Theorie) der Titelverbindung.
$R_f$ = 0,6 (Petrolether / Essigester 7:3)

Beispiel IX

N-Trifluoracetyl-L-prolinamid

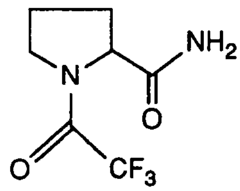

30 g (0,142 mol) Trifluoracetylprolin werden unter Schutzgas in 150 ml Dimethylformamid vorgelegt. Bei -20 °C gibt man 142,6 ml (0,1704 mol) 38 %iges Propanphosphorsäureanhydrid in Essigester zu. Es wird bis zur Sättigung Ammoniak eingeleitet, wobei nach 30 min ein weißer Niederschlag ausfällt. Unter schwachem Ammoniakstrom wird der Ansatz aufgetaut. Dann gibt man die gesamte Reaktionsmischung in 600 mi $H_2O$ und säuert mit konzentrierter Essigsäure bis pH 4 an. Es wird 4 x mit 200 ml Methylenchlorid und 3 x mit 200 ml Ether ausgeschüttelt. Die vereinigten organischen Phasen werden mit Magnesiumsulfat getrocknet und das Lösungsmittel abgezogen. Die Rückstände chromatographiert man zusammen über Kieselgel 60 F254, Methylenchlorid/Methanol (10:1). Die das Produkt enthaltenen Fraktionen werden am Rotationsverdampfer vom Lösungsmittel befreit.
Man erhält 17,12 g der Titelverbindung (57 % der Theorie);
$R_f$: 0,345 (Toluen/Essigester/$CH_3COOH$) 20:20:1.

Beispiel X

2-Cyano-N-trifluoracetyl-pyrrolidin

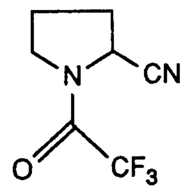

40 g (0,19 mol) des Produkts aus Beispiel IX und 45 g = 46 ml (0,57 mol) Pyridin legt man unter Schutzgas in 300 ml THF vor. Bei 0 °C tropft man 48 g = 32,25 ml (0,228 mol) Trifluoressigsäureanhydrid zu. Die Reaktionsmischung wird 30 min bei 0 °C und 90 min bei Raumtemperatur nachgerührt. Der Ansatz wird dann in 1 l 1N Salzsäure gegeben und 3 x mit 200 ml Methylenchlorid ausgeschüttelt. Die vereinigten organischen Phasen werden mit 200 ml gesättigter NaCl-Lösung ausgeschüttelt und über Magnesiumsulfat getrocknet Das Lösungsmittel wird abgezogen und der Rückstand wird an Kieselgel 60 F254 chromatographiert. Petrolether/Essigester/Essigsäure (1600:200:5). Die das Produkt enthaltenen Fraktionen engt man ein. Man erhält 32,4 g der Titelverbindung (88,8 % der Theorie).
$R_f$: 0,57 (Petroleumether/Essigester 7:3).

Beispiel XI

2-Tetrazolyl-N-trifluoracetyl-pyrrolidin

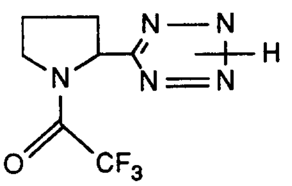

31,35 g = 32,6 ml (0,26 mol) Diethylaluminiumchlorid werden in 65 ml Toluol unter Schutzgas vorgelegt. Bei Raumtemperatur gibt man 29,95 g = 34,04 ml (0,26 mol) Trimethylsilylazid zu und es wird 10 min bei Raumtemperatur nachgerührt. Bei 0 °C gibt man 25 g (0,13 mol) des Produkts aus Beispiel X, gelöst in 65 ml Toluol, hinzu. Die Reaktionsmischung wird 30 min bei 0 °C, 120 min bei Raumtemperatur und 60 min bei 40 °C gerührt. Der abgekühlte Ansatz wird so lange mit gesättigter Kaliumfluorid-Lösung versetzt, bis keine Gasentwicklung mehr zu erkennen ist.

Die Reaktionsmischung wird in 600 ml $H_2O$ gegeben und bis pH 4 angesäuert und 3 x mit 100 ml Essigester extrahiert. Die vereinigten organischen Phasen werden mit 50 ml n-Hexan versetzt. Um die Azide zu entfernen, wird ca. 1/3 des Lösungsmittels über eine Destillationsbrücke ohne Kühlung abdestilliert. Der Rückstand wird über Magnesiumsulfat getrocknet und am Rotationsverdampfer vom Lösungsmittel befreit.
Man erhält 18,54 g der Titelverbindung (60,6 % der Theorie).
$R_f$: 0,4 (Toluol/Essigester 1:1).

Beispiel XII

N-Trifluoracetyl-2-[N-trityl-tetrazolyl]pyrrolidin

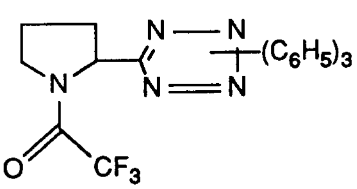

16,23 g (0,069 mol) des Produktes aus Beispiel XI und 10,47 g = 14,35 ml (0,1035 mol) Triethylamin werden in 70 ml Methylenchlorid vorgelegt. Dann gibt man 19,83 g (0,069 mol) Triphenylmethylchlorid zu. Man läßt die Reaktionsmischung 1,5 h bei Raumtemperatur nachrühren, verdünnt mit Methylenchlorid und extrahiert mit pH = 5 Pufferlösung (3 x 50 ml). Die organische Phase wird über Magnesiumsulfat getrocknet. Das Lösungsmittel wird am Rotationsverdampfer abgezogen. Der Rückstand wird mit Ether verrührt. Die entstandenen Kristalle werden abgesaugt und getrocknet.
Man erhält 24,65 g der Titelverbindung (75 % der Theorie).
$R_f$: 0,53 (Petrolether/Essigester 7:3).

Beispiel XIII

2-(N-Trityl-tetrazolyl)pyrrolidin

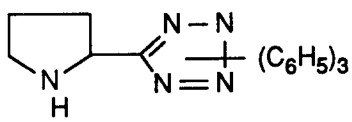

24 g (0,05 mol) des Produktes aus Beispiel XII werden unter Schutzgas in 100 ml Ethanol vorgelegt. Bei 0 ° C gibt man portionsweise 2,84 g (0,075 mol) Natriumborhydrid zu. Der Ansatz wird aufgetaut und 1 h bei Raumtemperatur gerührt. Man versetzt mit 6 ml Essigsäure und gibt die ganze Reaktionsmischung in 500 ml Pufferlösung pH 9. Der Ansatz wird mit 3 x 75 mi Methylenchlorid extrahiert. Die vereinigten organischen Phasen werden über Magnesiumsulfat getrocknet und am Rotationsverdampfer vom Lösungsmittel befreit. Der Rückstand wird an Kieselgel 60 F 254 chromatographiert. Petrolether/Essigester (7:3). Die entsprechenden Fraktionen werden eingeengt und getrocknet.
Man erhält 7,16 g der Titelverbindung (37,5 % der Theorie).
$R_f$: 0,22 (Essigester).

Beispiel XIV

4-Brommethyl-3-chlor-benzolsulfonsäure-2-[trityl-tetrazolyl]pyrrolidinid

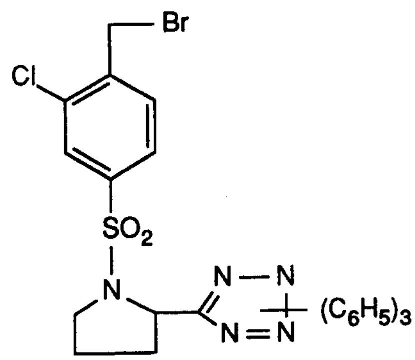

In Analogie zur Vorschrift des Beispiels III erhält man aus 3,19 g (10,5 mmol) der Verbindung aus Beispiel II und 4 g (10,5 mmol) der Verbindung aus Beispiel XIII 6,49 g der Titelverbindung (95 % der Theorie).
$R_f$: 0,53 (Petrolether/Essigester 7:3).

Beispiel XV

4-(Brommethyl)-3-fluorbenzolsulfochlorid

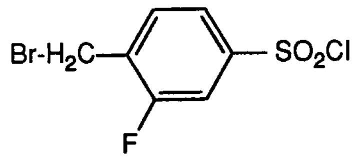

Man nimmt 20,9 g (0,1 mol) 3-Fluor-4-methylbenzolsulfochlorid in 200 ml Tetrachlorkohlenstoff auf und erhitzt nach Zugabe von 19,6 g (0,11 mol) N-Bromsuccinimid und 0,3 g Dibenzoylperoxid für 5 h unter Rückfluß. Nach dem Abkühlen werden die Feststoffe abfiltriert und das Filtrat vom Lösemittel befreit. Flashchromatographie Petrolether/Toluol (4:1), 50 µm Korngröße, ergibt 12,4 g (44 % der Theorie) der Titelverbindung.
$R_f$: 0,42 (Petrolether/Toluol 3:1).

Beispiel XVI

4-(Brommethyl)-3-trifluormethylbenzolsulfochlorid

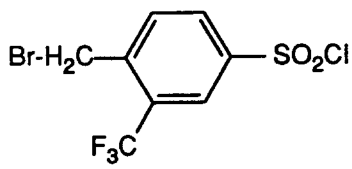

Man nimmt 64,6 g (0,25 mol) 3-Trifluormethyl-4-methylbenzolsulfochlorid in 500 ml Tetrachlorkohlenstoff auf und erhitzt nach Zugabe von 44,5 g (0,25 mol) N-Bromsuccinimid und 0,4 g ABN für 24 h unter Rückfluß. Nach dem Abkühlen werden die Feststoffe abfiltriert und das Filtrat vom Lösemittel befreit. Flashchromatographie Petrolether/Toluol (4:1), 50 um Korngröße, ergibt 33,9 g (40 % der Theorie) der Titelverbindung.
$R_f$: 0,41 (Petrolether/Toluol 3:1).

Beispiel XVII

(S)-4-(Brommethyl)-3-fluorbenzolsulfonyl-N-2-(tert.butoxy-carbonyl)pyrrolidinid

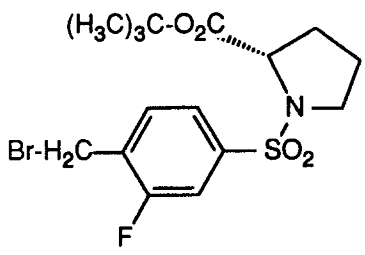

In Analogie zur Vorschrift aus Beispiel III erhält man aus 8,6 g (30 mmol) der Verbindung aus Beispiel XV und 5,1 g (30 mmol) S-Prolin-tert.-butylester 12,7 g (100 % der Theorie) der Titelverbindung.
$R_f$: 0,57 (Petrolether/Essigester 7:3).

Beispiel XVIII

(S)-4-(Brommethyl)-3-trifluormethylbenzolsulfonyl-N-2-(tert.butoxycarbonyl)-pyrrolidinid

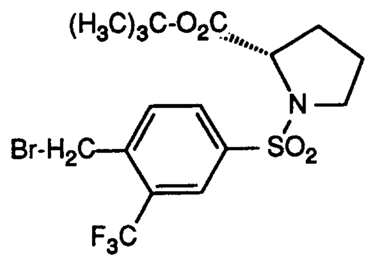

In Analogie zur Vorschrift des Beispiels III erhält man aus 16,9 g (50 mmol) der Verbindung aus Beispiel XVI und 8,6 g (50 mmol) S-Prolin-tert.-butylester 23,6 g (100 % der Theorie) der Titelverbindung.
$R_f$: 0,63 (Petrolether/Essigester 7:3).

Beispiel XIX

(S)-4-Carboxy-3-hydroxybenzolsulfonyl-N-2(tert.-butoxycarbonyl)pyrrolidinid

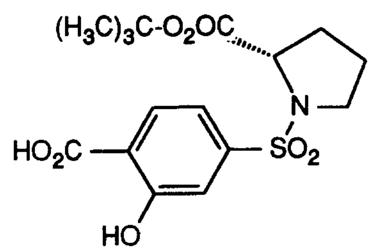

In Analogie zur Vorschrift des Beispiels III erhält man aus 23,7 g 4-Carboxy-3-hydroxybenzolsulfochlorid (100 mmol) 17,1 g (100 mmol) S-Prolin-tert.-butylester 30,0 g (81 % der Theorie) der Titelverbindung.
$R_f$: 0,18 (Aceton)

Beispiel XX

(S)-4-Benzoyloxycarbonyl-3-benzyloxybenzolsulfonsäure-N-2-(tert.-butoxycarbonyl)pyrrolidinid

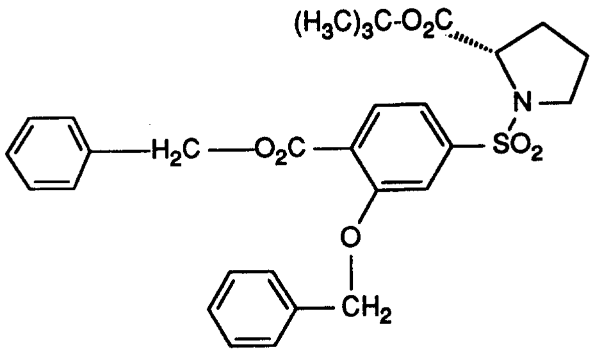

25,3 g (68 mmol) der Verbindung aus Beispiel XIX gelöst in 200 ml DMF werden mit 28,3 g $K_2CO_3$ - (204 mmol) und 25,7 g (150 mmol) Benzylbromid versetzt. Man läßt die Reaktionsmischung 2 Stunden bei 75 °C nachrühren, gibt nach dem Abkühlen 1 l Wasser hinzu, extrahiert mit Essigester (3 x 400 ml), wäscht mit Wasser (5 x 400 ml), trocknet über $MgSO_4$, filtriert und zieht alles Flüchtige im Vakuum ab. Man reinigt durch Flash-Chromatographie (Petrolether/$CH_2Cl_2$ 5:1 und Petrolether/Essigester 6:1), 50 um Teilchengröße. Zur weiteren Reinigung wird das Produkt aus 600 ml eines Lösungsmittelgemisches (Petrolether/Essigester 6:1) umkristallisiert und man erhält 35,5 g (95 % der Theorie) der Titelverbindung.
$R_f$ = 0,53 (Petrolether/Essigester 7:3).

Beispiel XXI

(S)-4-(Hydroxymethyl)-3-benzyloxybenzolsulfonsäure-N-2-(tert.butoxycarbonyl)-pyrrolidinid

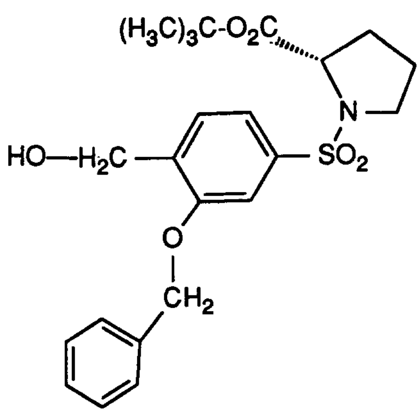

11,03 g (20 mmol) der Verbindung aus Beispiel XX werden in 100 ml Diglycene gelöst und nach Zugabe von 1,51 g (40 mmol) Natriumborhydrid und 1,68 g (40 mmol) LiCl für 4 Stunden bei 70 °C nachgerührt. Nach dem Abkühlen wird das Reaktionsgemisch mit 500 ml Wasser versetzt und mit 1 H HCl bis pH 3 angesäuert. Es wird mit Ether extrahiert (3 x 300 ml), mit Wasser gewaschen (6 x 300 ml), über $MgSO_4$ getrocknet und das Filtrat vom Lösemittel befreit. Der Rückstand wird an Kieselgel 60 F 254 chromatographiert. Petrolether/Essigester (7:3). Die entsprechenden Fraktionen werden eingeengt und getrocknet. Man erhält 5,0 g (56 % der Theorie) der Titelverbindung.
$R_f$: 0,36 (Petrolether/Essigester 7:3).

Beispiel XXII

(S)-4-(Brommethyl)-3-benzyloxybenzolsulfonsäure-N-2-(tert.butoxycarbonyl)-pyrrolidinid

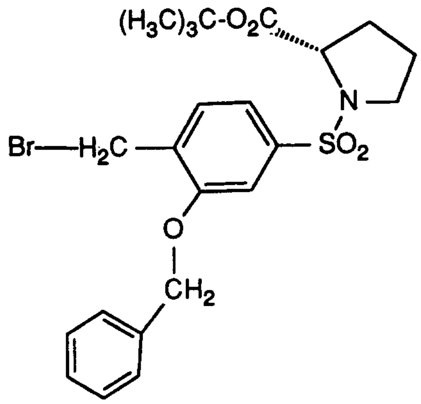

2,24 g (5 mmol) der Verbindung aus Beispiel XXI werden unter Schutzgas in 20 ml absolutem DMF vorgelegt. Bei 0 °C gibt man 2,53 g (6 mmol) Triphenylphosphin-dibromid hinzu. Das Reaktionsgemisch wird 1 Stunde bei Raumtemperatur gerührt. Man versetzt mit 200 ml Wasser, extrahiert mit Essigester (3 x 80 ml), wäscht mit Wasser (5 x 60 ml), trocknet über $MgSO_4$, filtriert und zieht alles Flüchtige im Vakuum ab. Man reinigt durch Flash-Chromatogaphie ($CH_2Cl_2$, 50 $\mu$m Teilchengröße) und erhält 2,55 g (100 % der Theorie) der Titelverbindung.
$R_f$: 0,56 (Petrolether/Essigester 7:3).

Herstellungsbeispiele

Beispiel 1

rac-4-[[2-Butyl-imidazo[4,5-b]pyridin]-3-yl]methyl-3-chlorbenzolsulfonyl-N-(2-tert.-butoxycarbonyl)piperidinid

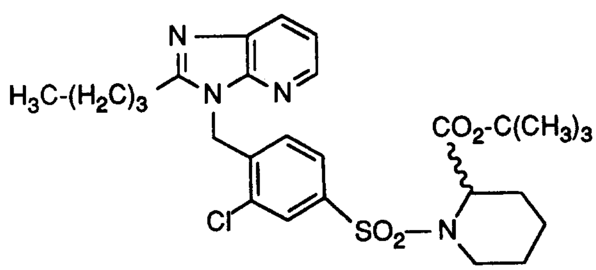

2,3 g (5 mmol) der Verbindung aus Beispiel VIII gelöst in 8 ml DMF gibt man zu einer Lösung von 613 mg (3,5 mmol) 2-Butylimidazo[4,5-b]pyridin und 105 mg (3,5 mmol) Natriumhydrid und rührt 3 h bei 20 °C nach. Man gibt die Reaktionsmischung auf Eis, extrahiert mit Essigester (3 x 50 ml), wäscht mit gesättigter NaCl-Lösung (5 x 50 ml), trocknet über $MgSO_4$, filtriert und zieht alles Flüchtige im Vakuum ab. Man reinigt durch Flash-Chromatographie ($CH_2Cl_2$/Essigester 20:1, 50$\mu$ Teilchengröße) und chromatographiert das erhaltene Produkt anschließend über Kieselgel (Essigester / Petrolether 1:1, 50 $\mu$ Teilchengröße) und erhält 563 mg (30% der Theorie) der Titelverbindung.
$R_F$ = 0,72 (Essigester / Petrolether 2:1)

Beispiel 2

rac-4-[(2-Butyl-imidazo[4,5-b]pyridin]-3-yl]methyl-3-chlorbenzolsulfonyl-N-(2-carboxy)piperidinid

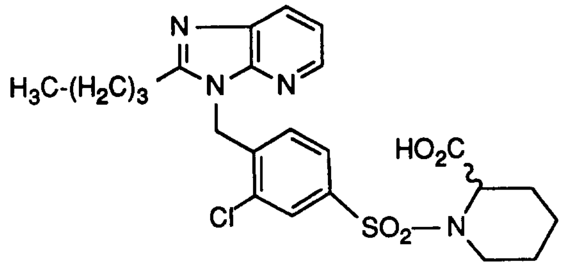

273 mg (0,5 mmol) der Verbindung aus Beispiel 1 werden in 10 ml Dichlormethan gelöst und mit 2 ml Trifluoressigsäure versetzt Man rührt 3 h bei 20 °C nach und zieht alles Flüchtige im Vakuum ab. Der Rückstand wird in 20 mi Dichlormethan / Ether 1:1 aufgenommen und anschließend bis zur Trockene eingeengt. Dieser Prozeß wird dreimal wiederholt und man erhält 300 mg (99% der Theorie) der Titelverbindung als Trifluoracetat-Salz.

$R_f$ = 0,30 ($CH_2Cl_2$/ MeOH 10:1)

In Analogie zu den oben aufgeführten Verfahren werden die in den Tabellen 1, 2 und 3 aufgeführten Verbindungen hergestellt:

Tabelle 1:

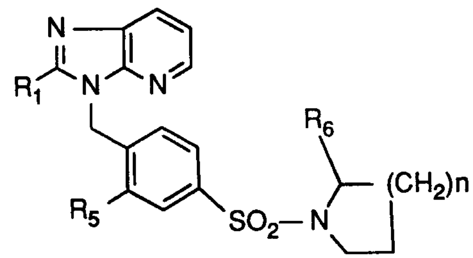

| Bsp.-Nr. | $R^1$ | $R^5$ | $R^6$ | n | Konfig. | $R_f^*$ | Ausbeute [%] |
|---|---|---|---|---|---|---|---|
| 3 | $-(CH_2)_3CH_3$ | H | H | 2 | - | 0,13 [b] | 29,3 |
| 4 | $-(CH_2)_3CH_3$ | Cl | $-CO_2C(CH_3)_3$ | 1 | S | 0,72 [c] | 24,3 |
| 5 | $-(CH_2)_3CH_3$ | Cl | $-CO_2H$ | 1 | S | 0,18 [a] | 24,0 |
| 6 | $-(CH_2)_3CH_3$ | H | $-CO_2C(CH_3)_3$ | 1 | S | 0,20 [d] | 30,0 |
| 7 | $-(CH_2)_3CH_3$ | H | $-CO_2C(CH_3)_3$ | 2 | rac | 0,23 [d] | 24,0 |
| 8 | $-(CH_2)_3CH_3$ | H | $-CO_2H$ | 1 | S | 0,16 [a] | 96,2 |
| 9 | $-(CH_2)_3CH_3$ | H | $-CO_2H$ | 2 | rac | 0,22 [a] | 99,2 |

Tabelle 2:

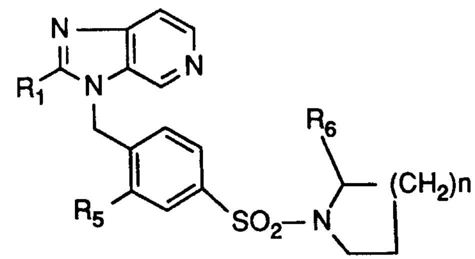

| Bsp.-Nr. | $R^1$ | $R^5$ | $R^6$ | n | Konfig. | $R_f^*$ | Ausbeute [%] |
|---|---|---|---|---|---|---|---|
| 10 | $-(CH_2)_3CH_3$ | H | H | 1 | - | 0,14 [a] | 6,6 |

Tabelle 3:

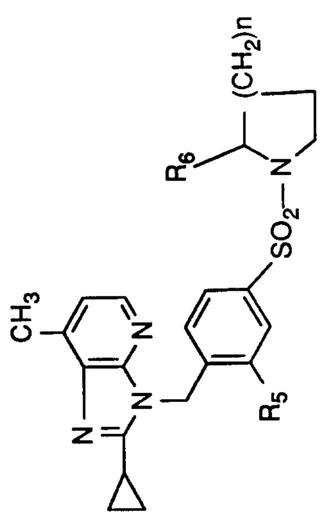

| Bsp.-Nr. | $R^5$ | $R^6$ | n | Konfig. | $R_f^*$ | Ausbeute [%] |
|---|---|---|---|---|---|---|
| 11 | H | $-CO_2C(CH_3)_3$ | 1 | S | 0,64 [a] | 26,5 |
| 12 | Cl | $-CO_2C(CH_3)_3$ | 1 | S | 0,77 [a] | 35,3 |
| 13 | H | $-CO_2C(CH_3)_3$ | 2 | rac | 0,75 [a] | 28,9 |
| 14 | Cl | $-CO_2C(CH_3)_3$ | 2 | rac | 0,81 [a] | 30,2 |
| 15 | H | $-CO_2H$ | 1 | S | 0,26 [d] | 99,3 |
| 16 | Cl | $-CO_2H$ | 1 | S | 0,40 [d] | 99,5 |
| 17 | H | $-CO_2H$ | 2 | rac | 0,58 [d] | 99,6 |
| 18 | Cl | $-CO_2H$ | 2 | rac | 0,68 [d] | 99,5 |
| 19 | Cl | $-CO_2CH_3$ | 1 | S | 0,42 [a] | 84,9 |

Beispiel 20

S-4-[[2-Ethyl-imidazo[4.5-b]5,7-dimethyl-pyridin]-3-yl]methyl-3-chlor-benzolsulfonyl-N-(2-methoxycarbonyl)-pyrrolidinid

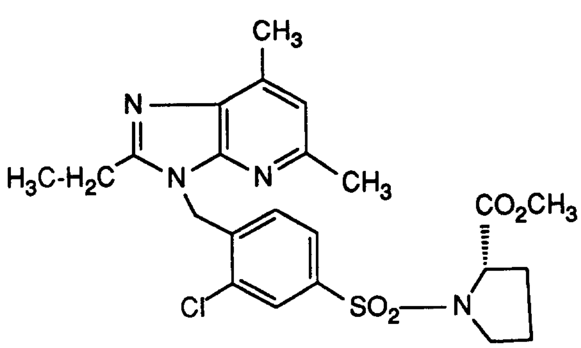

296 mg (0,5 mmol) der Verbindung aus Beispiel 29 (siehe Tabelle 4), gelöst in 5 ml DMF, werden nacheinander mit 345 mg (2,5 mmol) Kaliumcarbonat und 99 mg (0,7 mmol) Jodmethan versetzt. Man rührt noch 10 min bei 20 °C, versetzt das Reaktionsgemisch mit 30 ml Wasser und 3 ml 1N HCl. Es wird mit Essigester extrahiert (3 x 50 ml), wäscht die vereinigten organischen Phasen mit Wasser (5 x 50 ml), trocknet über $MgSO_4$, filtriert und zieht alles Flüchtige im Vakuum ab. Man erhält 211 mg (86 % der Theorie) der Titelverbindung.
$R_f$: 0,67 (Dichlormethan/Methanol 10:1)
Konfiguration: S.

Beispiel 21 und 22

S-4-[[2-Butyl-imidazo[4.5b]5,7-dimethyl-pyridin]-3-yl]methyl-3-chlor-benzolsulfonyl-N-(2-carboxy)pyrrolidinid (Beispiel 21)

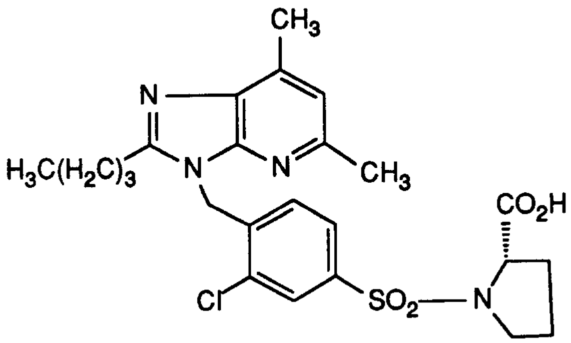

(21)

1,0 g (1,62 mmol) der Verbindung aus Beispiel 47 (siehe Tabelle 4) werden mit 300 ml pH 7 Puffer-Lösung und 150 ml Chloroform 0,5 h bei 20 °C kräftig gerührt. Die organische Phase wird abgetrennt, die wäßrige Phase wird mit Chloroform extrahiert (4 x 50 ml). Man wäscht die vereinigten organischen Phasen mit Wasser (2 x 100 ml), trocknet über $MgSO_4$, filtriert und zieht alles Flüchtige im Vakuum ab. Nach dem Trocknen im Vakuum erhält man 0,70 g (86 % der Theorie) der Titelverbindung (Beispiel 21). Konfiguration: S

S-4-[[2-Butyl-imidazo[4,5b]-5,7-dimethyl-pyridin]-3-yl]methyl-3-chlor-benzolsulfonyl-N-(2-carboxy)-pyrrolidinid-Kaliumsalz (Beispiel 22)

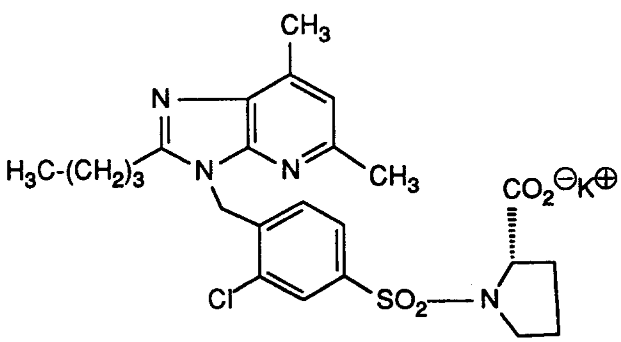

(22)

334 mg (0,66 mmol) der Verbindung aus Beispiel 21, gelöst in 5 ml Methanol, werden mit 43 mg (0,66 mol) 85 %igem Kaliumhydroxid-Pulver versetzt. Die flüchtigen Bestandteile werden im Vakuum abgezogen. Nach dem Trocknen erhält man 359 mg (100 % der Theorie) der Titelverbindung als Kaliumsalz. (Beispiel 22) Konfiguration: S.

Die Verbindungen in Tabelle 4 werden in Analogie zu den Vorschriften der Beispiele 20, 21 und 22 hergestellt.

Tabelle 4:

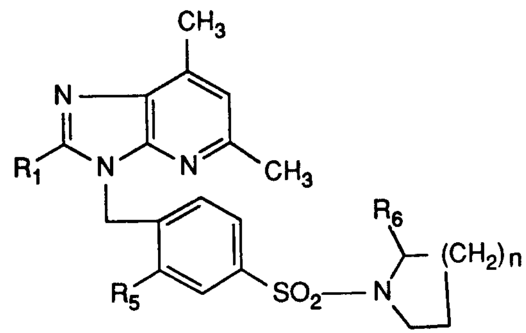

| Beisp. Nr. | $R^1$ | $R^5$ | $R^6$ | n | Konfig. | $R_f$ | Ausbeute (% der Th.) | Salz |
|---|---|---|---|---|---|---|---|---|
| 23 | 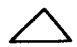 | Cl | $-CO_2C(CH_3)_3$ | 1 | S | 0,59[a] | 83,0 | |
| 24 | 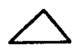 | $CF_3$ | $-CO_2C(CH_3)_3$ | 1 | S | 0,65[a] | 83,5 | |
| 25 | 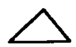 | Cl | $-CO_2H$ | 1 | S | 0,13[a] | 100 | TFA*-Salz |
| 26 | 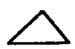 | $CF_3$ | $-CO_2H$ | 1 | S | 0,16[a] | 100 | TFA*-Salz |
| 27 | $C_2H_5$ | Cl | $-CO_2C(CH_3)_3$ | 1 | S | 0,69[a] | 76,5 | |
| 28 | $C_2H_5$ | $CF_3$ | $-CO_2C(CH_3)_3$ | 1 | S | 0,74[a] | 79,5 | |
| 29 | $C_2H_5$ | Cl | $-CO_2H$ | 1 | S | 0,25[a] | 100 | TFA*-Salz |
| 30 | $C_2H_5$ | $CF_3$ | $-CO_2H$ | 1 | S | 0,28[a] | 100 | TFA*-Salz |
| 31 | 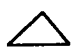 | Cl | $-CO_2CH_3$ | 1 | S | 0,57[a] | 85,7 | |
| 32 | 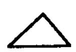 | Cl | $-CO_2-CH_2C_6H_5$ | 1 | S | 0,65[a] | 68,5 | |
| 33 | $C_2H_5$ | Cl | $-CO_2-CH_2C_6H_5$ | 1 | S | 0,79[a] | 59,0 | |

Tabelle 4: (Fortsetzung)

| Beisp. Nr. | $R^1$ | $R^5$ | $R^6$ | n | Konfig. | $R_f$ | Ausbeute (% der Th.) | Salz |
|---|---|---|---|---|---|---|---|---|
| 34 | 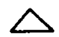 | F | $-CO_2C(CH_3)_3$ | 1 | S | 0,69[a] | 86,4 | |
| 35 | 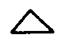 | F | $-CO_2H$ | 1 | S | 0,22[a] | 100 | TFA*-Salz |
| 36 | 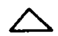 | F | $-CO_2CH_3$ | 1 | S | 0,77[a] | 85,7 | |
| 37 | 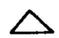 | F | $-CO_2CH_2C_6H_5$ | 1 | S | 0,84[a] | 72,5 | |
| 38 | 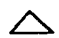 | Cl | $-CO_2H$ | 1 | S | | 93,4 | |
| 39 | 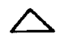 | Cl | $-CO_2H$ | 1 | S | | 94,9 | K**-Salz |
| 40 | $-C_2H_5$ | Cl | $-CO_2H$ | 1 | S | | 76,9 | |
| 41 | $-C_2H_5$ | Cl | $-CO_2H$ | 1 | S | | 96,7 | K**-Salz |
| 42 | 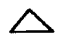 | Cl | $-CO_2CH_3$ | 1 | S | | 100 | TFA*-Salz |
| 43 | $-C_2H_5$ | Cl | $-CO_2CH_3$ | 1 | S | | 100 | TFA*-Salz |
| 44 | $-C_2H_5$ | Cl | $-CO_2-CH_2C_6H_5$ | 1 | S | | 100 | TFA*-Salz |
| 45 | 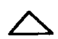 | Cl | $-CO_2-CH_2C_6H_5$ | 1 | S | | 100 | TFA*-Salz |
| 46 | $n-C_4H_9$ | Cl | $-CO_2C(CH_3)_3$ | 1 | S | 0,26[e] | 85,9 | |
| 47 | $n-C_4H_9$ | Cl | $-CO_2H$ | 1 | S | 0,13[a] | 100 | TFA*-Salz |
| 48 | 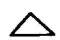 | Cl | 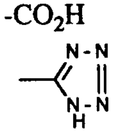 | 1 | S | 0,07[e] | 24,8 | |
| 49 | 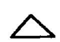 | Cl | 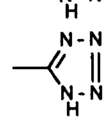 | 1 | S | | 98,8 | |

* TFA = Trifluoressigsäure
** K = Kalium

Beispiel 50

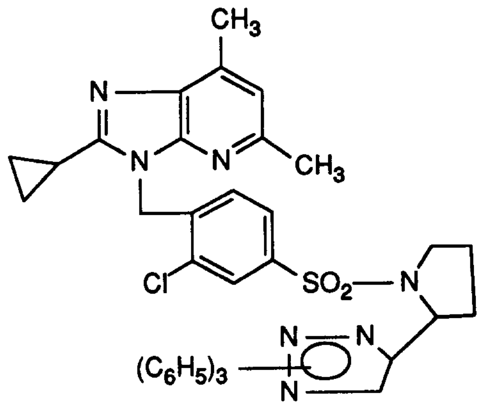

Analog zu Vorschrift für Beispiel 1 erhält man aus 3 g (4,62 mmol) der Verbindung aus Beispiel XIV 2,08 g (60 % der Theorie) der Titelverbindung.

$R_f$ = 0,46 (Petrolether/Essigester 1:1).

In Analogie zu den Vorschriften der Beispiele 1 und 2 werden die in Tabelle 5 aufgeführten Verbindungen hergestellt.

Tabelle 5:

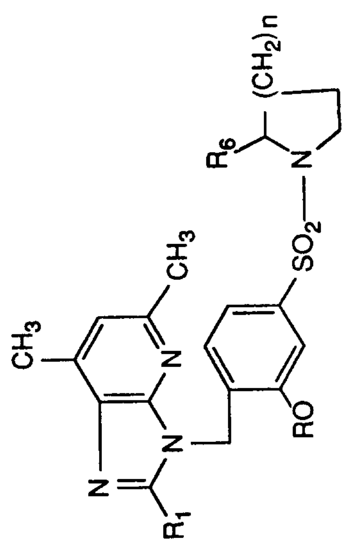

| Beisp. Nr. | $R^1$ | $R^5$ | $R^6$ | n | Konfig. | $R_f$ | Ausbeute (% der Th.) |
|---|---|---|---|---|---|---|---|
| 51 | 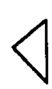 | $-O-CH_2-$ 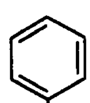 | $-CO_2C(CH_3)_3$ | 1 | S | 0,19[g] | 86 |
| 52 | 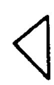 | $-O-CH_2-$ 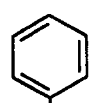 | $-CO_2H$ | 1 | S | 0,17[a] | 98 |
| 53 | 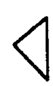 | -H | $-CO_2H$ | 1 | S | 0,26[f] | 53 |
| 54 | 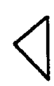 | -H | $-CO_2C(CH_3)_3$ | 1 | S | 0,23[b] | 89 |

## Patentansprüche

1. Sulfonylbenzyl-substituierte Imidazopyridine der allgemeinen Formel

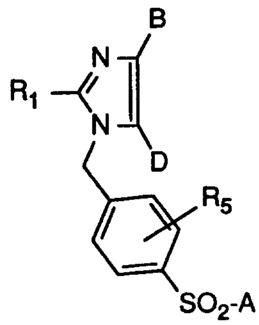

(I),

in welcher

$R^1$ für geradkettiges oder verzweigtes Alkyl oder Alkenyl mit jeweils bis zu 8 Kohlenstoffatomen steht, die gegebenenfalls durch Cycloalkyl mit 3 bis 6 Kohlenstoffatomen substituiert sind, oder
für Cycloalkyl mit 3 bis 8 Kohlenstoffatomen steht,

B und D gemeinsam einen heterocyclischen Rest der Formel

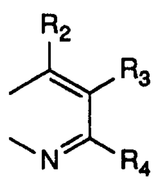

,

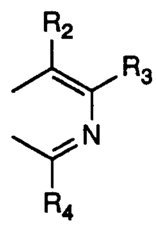

,

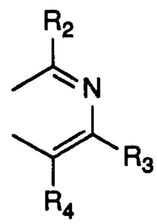

oder

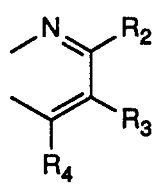

bilden,
worin

$R^2$ und $R^3$ gleich oder verschieden sind und Wasserstoff, Halogen oder geradkettiges oder verzweigtes Alkyl mit bis zu 8 Kohlenstoffatomen bedeuten,

$R^4$ die oben angegebene Bedeutung von $R^2$ und $R^3$ hat und mit diesen gleich oder verschieden ist,
oder eine Gruppe der Formel $-CO-R^6$ bedeutet,
worin

$R^6$ Hydroxy, geradkettiges oder verzweigtes Alkoxy mit bis zu 8 Kohlenstoffatomen, Phenoxy, Benzyloxy oder eine Gruppe der Formel $-NR^7R^8$ bedeutet,
worin

$R^7$ und $R^8$ gleich oder verschieden sind und Wasserstoff oder geradkettiges oder verzweigtes Alkyl mit bis zu 6 Kohlenstoffatomen oder Phenyl bedeuten,

$R^5$ für Wasserstoff, Halogen, geradkettiges oder verzweigtes Alkyl mit bis zu 8 Kohlenstoffatomen steht, oder
für geradkettiges oder verzweigtes Perfluoralkyl mit bis zu 6 Kohlenstoffatomen steht,
oder
für eine Guppe der Formel -OX steht,
worin

X Wasserstoff, Benzyl, eine Hydroxyschutzgruppe oder geradkettiges oder verzweigtes Alkyl mit bis zu 8 Kohlenstoffatomen bedeutet,

A für einen über das Stickstoffatom gebundenen 3 bis 8-gliedrigen, gesättigten Heterocyclus mit bis zu 2 weiteren Heteroatomen aus der Reihe S, H oder O steht und der gegebenenfalls bis zu 2-fach gleich oder verschieden durch Perfluoralkyl mit bis zu 5 Kohlenstoffatomen oder durch einen Rest der Formel

$-SO_3H$ ,

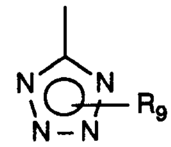 ,

$-CO-R^{10}$ oder

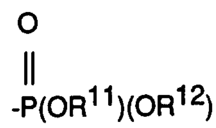

substituiert ist,

worin

$R^9$ Wasserstoff, geradkettiges oder verzweigtes Alkyl mit bis zu 6 Kohlenstoffatomen oder Triphenylmethyl bedeutet

$R^{10}$ die oben angegebene Bedeutung von $R^6$ hat und mit diesem gleich oder verschieden ist

und

$R^{11}$ und $R^{12}$ gleich oder verschieden sind und Wasserstoff, geradkettiges oder verzweigtes Alkyl mit bis zu 8 Kohlenstoffatomen oder Phenyl bedeuten,

und deren Salze.

**2.** Sulfonylbenzyl-substituierte Imidazopyridine nach Anspruch 1, wobei

$R^1$ für geradkettiges oder verzweigtes Alkyl oder Alkenyl mit jeweils bis zu 6 Kohlenstoffatomen steht, die gegebenenfalls durch Cyclopropyl, Cyclobutyl, Cyclopentyl oder Cyclohexyl substituiert sind, oder

für Cyclopropyl, Cyclopentyl oder Cyclohexyl steht,

B und D gemeinsam einen heterocyclischen Rest der Formel

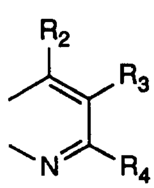

oder

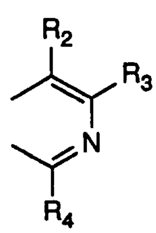

bilden,

worin

$R^2$ und $R^3$ gleich oder verschieden sind und Wasserstoff, Fluor, Chlor, Brom, geradkettiges oder verzweigtes Alkyl mit bis zu 6 Kohlenstoffatomen bedeuten,

$R^4$ die oben angegebene Bedeutung von $R^2$ und $R^3$ hat und mit diesen gleich oder verschieden ist,

oder eine Gruppe der Formel $-CO-R^6$ bedeutet,

worin

$R^6$ Hydroxy, geradkettiges oder verzweigtes Alkoxy mit bis zu 6 Kohlenstoffatomen, Phenoxy, Benzyloxy oder eine Gruppe der Formel $-NR^7R^8$ bedeutet,

worin

$R^7$ und $R^8$ gleich oder verschieden sind und Wasserstoff oder geradkettiges oder verzweigtes Alkyl mit bis zu 4 Kohlenstoffatomen bedeuten,

$R^5$ für Wasserstoff, Fluor, Chlor, Brom, geradkettiges oder verzweigtes Alkyl mit bis zu 6 Kohlenstoffatomen, oder

für geradkettiges oder verzweigtes Perfluoralkyl mit bis zu 4 Kohlenstoffatomen steht,

oder

für eine Guppe der Formel -OX steht,

worin

X Wasserstoff, Benzyl, Acetyl oder geradkettiges oder verzweigtes Alkyl mit bis zu 6 Kohlenstoffatomen bedeutet,

A für über das Stickstoffatom gebundenes Azetidinyl, Piperidyl, Pyrrolidinyl oder Morpholinyl steht, die gegebenenfalls durch Trifluormethyl oder durch einen Rest der Formel

$-SO_3H$ ,

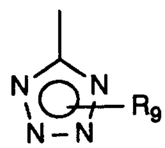 ,

$-CO-R^{10}$ oder

$$\overset{O}{\overset{\|}{-P}}(OR^{11})(OR^{12})$$

substituiert sind,

worin

$R^9$ Wasserstoff, geradkettiges oder verzweigtes Alkyl mit bis zu 4 Kohlenstoffatomen oder Triphenylmethyl bedeutet

$R^{10}$ die oben angegebene Bedeutung von $R^6$ hat und mit diesem gleich oder verschieden ist

und

$R^{11}$ und $R^{12}$ gleich oder verschieden sind und Wasserstoff, geradkettiges oder verzweigtes Alkyl mit bis zu 6 Kohlenstoffatomen oder Phenyl bedeuten,

und deren Salze.

**3.** Sulfonylbenzyl-substituierte Imidazopyridine nach Anspruch 1, wobei

$R^1$ für geradkettiges oder verzweigtes Alkyl oder Alkenyl mit jeweils bis zu 4 Kohlenstoffatomen oder Cyclopropyl steht,

B und D gemeinsam einen heterocyclischen Rest der Formel

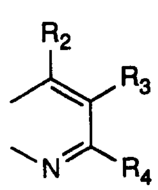

oder

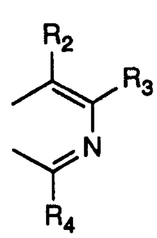

bilden,
worin

$R^2$ und $R^3$ gleich oder verschieden sind und Wasserstoff, Fluor, Chlor, Brom oder geradkettiges oder verzweigtes Alkyl mit bis zu 4 Kohlenstoffatomen bedeuten,

$R^4$ die oben angegebene Bedeutung von $R^2$ und $R^3$ hat und mit diesen gleich oder verschieden ist,
oder eine Gruppe der Formel $-CO-R^6$ bedeutet,
worin

$R^6$ Hydroxy, geradkettiges oder verzweigtes Alkoxy mit bis zu 4 Kohlenstoffatomen, Phenoxy, Benzyloxy oder eine Gruppe der Formel $-NR^7R^8$ bedeutet,
worin

$R^7$ und $R^8$ gleich oder verschieden sind und Wasserstoff oder geradkettiges oder verzweigtes Alkyl mit bis zu 3 Kohlenstoffatomen bedeuten,

$R^5$ für Wasserstoff, Fluor, Chlor, geradkettiges oder verzweigtes Alkyl mit bis zu 4 Kohlenstoffatomen, oder
für geradkettiges oder verzweigtes Perfluoralkyl mit bis zu 3 Kohlenstoffatomen steht,
oder
für eine Guppe der Formel -OX steht,
worin

X Wasserstoff, Benzyl, Acetyl oder geradkettiges oder verzweigtes Alkyl mit bis zu 6 Kohlenstoffatomen bedeutet,

A für über das Stickstoffatom gebundenes Azetidinyl, Piperidyl oder Pyrrolidinyl steht, die gegebenenfalls durch Trifluormethyl oder durch einen Rest der Formel
$-SO_3H$ ,

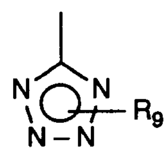 ,

$-CO-R^{10}$ oder

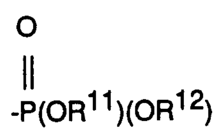

substituiert sind,
worin

$R^9$ Wasserstoff, Methyl, Ethyl oder Triphenylmethyl bedeutet

$R^{10}$ die oben angegebene Bedeutung von $R^6$ hat und mit diesem gleich oder verschieden ist
und

$R^{11}$ und $R^{12}$ gleich oder verschieden sind und Wasserstoff, geradkettiges oder verzweigtes Alkyl mit bis zu 4 Kohlenstoffatomen oder Phenyl bedeuten,

und deren Salze.

**4.** Sulfonylbenzyl-substituierte Imidazopyridine nach Anspruch 1 zur therapeutischen Anwendung.

**5.** Verfahren zur Herstellung von Sulfonylbenzyl-substituierten Imidazopyridinen nach Anspruch 1, dadurch gekennzeichnet, daß man Sulfonylbenzylverbindungen der allgemeinen Formel (II)

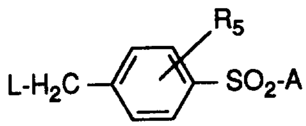 (II)

in welcher

A und $R^5$ die in Anspruch 1 angegebene Bedeutung haben

und

L für Halogen, vorzugsweise für Brom steht,

mit Imidazopyridinen der allgemeinen Formel (III)

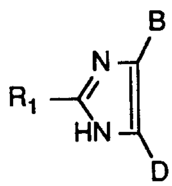 (III)

in welcher

$R^1$, B und D die in Anspruch 1 angegebene Bedeutung haben,

in inerten Lösemitteln, in Anwesenheit einer Base umsetzt,

und

sowohl die Substituenten $R^1$ und $R^5$, als auch die der heterocyclischen Ringe (A, B und D), gegebenenfalls nach üblichen Methoden, beispielsweise durch Alkylierung oder Verseifung, variiert.

6. Arzneimittel enthaltend mindestens ein Sulfonylbenzyl-substituiertes Imidazopyridin nach Anspruch 1.

7. Verfahren zur Herstellung von Arzneimitteln nach Anspruch 6, dadurch gekennzeichnet, daß man die Sulfonylbenzyl-substituierten Imidazopyridine gegebenenfalls mit Hilfs- und Trägerstoffen in eine geeignete Applikationsform bringt.

8. Verwendung von Sulfonylbenzyl-substituierten Imidazopyridinen nach Anspruch 1 zur Herstellung von Arzneimitteln.

9. Sulfonylbenzylverbindungen der allgemeinen Formel

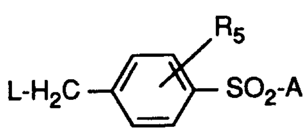 (II)

wobei

$R^5$ und A die angegebene Bedeutung haben

und

L für Halogen steht.

10. Verfahren zur Herstellung von Sulfonylbenzylverbindungen der allgemeinen Formel

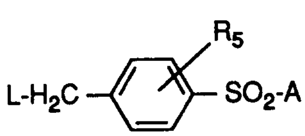 (II)

wobei

$R^5$, A und L die angegebene Bedeutung haben,

dadurch gekennzeichnet, daß man Verbindungen der allgemeinen Formel (IV)

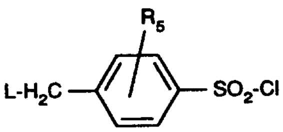

(IV)

in welcher

L und $R^5$ die oben angegebene Bedeutung haben,

mit Verbindungen der allgemeinen Formel (V)

A-H (V)

in welcher

A die oben angegebene Bedeutung hat,

in einem inerten organischen Lösemittel in Anwesenheit einer Base umsetzt.